(19) Europäisches Patentamt
European Patent Office
Office européen des brevets

(11) Publication number: **0 072 137**
**A1**

# (12) EUROPEAN PATENT APPLICATION

(21) Application number: **82303933.4**

(22) Date of filing: **26.07.82**

(51) Int. Cl.³: **C 07 H 19/06**, A 61 K 31/70

(30) Priority: **01.08.81 GB 8123601**

(43) Date of publication of application: **16.02.83**
***Bulletin 83/7***

(84) Designated Contracting States: **BE CH DE FR GB IT LI NL SE**

(71) Applicant: **BEECHAM GROUP PLC, Beecham House Great West Road, Brentford Middlesex (GB)**

(72) Inventor: **Harnden, Michael Raymond, 47 Guildford Road, Horsham Sussex (GB)**
Inventor: **Shanks, Colin Thomas, 12 Meath Green Avenue, Horley Surrey (GB)**

(74) Representative: **Russell, Brian John et al, European Patent Attorney Beecham Pharmaceuticals Great Burgh Yew Tree Bottom Road, Epsom Surrey, KT18 5XQ (GB)**

(54) **Antiviral deoxyuridine compounds.**

(57) A compound of formula (I):

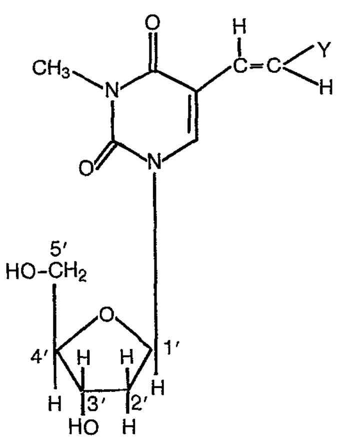

(I)

or a pharmaceutically acceptable active ester thereof, in which Y is a halogen atom,
is useful in the treatment of infections caused by herpes viruses.

EP 0 072 137 A1

TITLE MODIFIED
see front page

NOVEL COMPOUNDS

This invention relates to certain deoxyuridine compounds which have antiviral activity.

UK Patent Specification No. 1601020 discloses 5-(2-halogenovinyl)-2'-deoxyuridines which have antiviral activity selective against herpes virus.

We have now found a group of methyl substituted derivatives of 5-(2-halogenovinyl)-2'-deoxyuridine which have excellent antiviral activity, and which are useful in the treatment of infections caused by herpes viruses, such as herpes simplex type 1, herpes simplex type 2 and varicella.

The derivatives give surprisingly prolonged high levels of antiviral activity in the blood of animals when administered orally.

According to the present invention there is provided a compound of the formula (I) or a pharmaceutically acceptable active ester thereof:

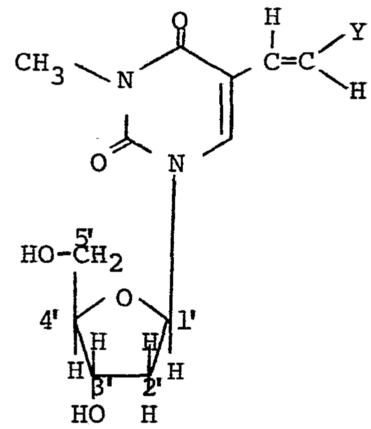

(I)

in which Y is a halogen atom, preferably a bromine atom.

The compounds of formula (I) may be prepared by treating a 5-(2-halogenovinyl)-2'-deoxyuridine compound of formula (II):

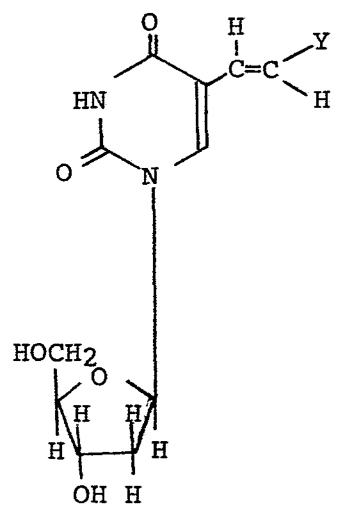

(II)

in which Y is as defined in formula (I) with a methyl halide of formula $CH_3X$, in which X is a halogen atom, preferably an iodine atom.

The reaction is suitably carried out in a polar organic solvent, preferably anhydrous tetrahydrofuran,

at room temperature, and is preferably catalysed by fluoride ions.

The product is preferably purified chromatographically by, for example, column chromatography on silica gel.

An alternative process for preparing the compounds of formula (I) comprises treating a compound of formula (II) as defined above with N,N-dimethyl formamide dimethylacetal, preferably in the presence of an acid catalyst such as trifluoroacetic acid.

The process is suitably carried out under an inert atmosphere, preferably nitrogen, by heating under reflux. The product may be obtained by heating the reaction mixture to dryness, and purifying chromatographically on silica gel.

Esters of the compounds of formula (I) may be 3',5'- diesters, or 3'-mono or 5'-mono esters, and these may be prepared by acylating the compounds of formula (I) with an appropriate acylating agent, such as organic acid chloride or anhydride, under normal acylating conditions.

The compounds of formula (I) or esters thereof may be formulated for use in a pharmaceutical composition. Accordingly, in a further aspect of the invention, there is provided a pharmaceutical composition which comprises a compound of the formula (I) or ester thereof together with a pharmaceutically acceptable carrier or excipient.

Compositions which may be given by the oral route may be compounded in the form of syrups, tablets and capsules. When the composition is in the form of a tablet, any pharmaceutical carrier suitable for

formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the compounds of the invention may be made up into a cream, lotion or ointment. These formulations may be conventional formulations well known in the art, for example, as described in standard books of pharmaceutics and cosmetics, such as Harry's Cosmeticology published by Leonard Hill Books, and the British Pharmacopaeia.

The composition for application to the eyes may be a conventional eye-drop composition well known in the art.

Preferably, the compositions of this invention are in unit dosage form or in some other form that the patient may administer to himself a single dose. A suitable dosage unit might contain from 50 mg to 1 g of active ingredient, for example 100 to 500 mg. Such doses may be adminstered 1 to 4 times a day or more usually 2 or 3 times a day. The effective dose of compound depends on the particular compound employed, but is in general in the range of from 1.0 mg/kg/day to 20 mg/kg of body weight per day or more usually 2.0 mg/kg/day to 10 mg/kg/day.

Accordingly, in a further aspect of the invention, there is provided a method of treating viral infections in human or non-human animals which comprises administering to the animal an effective amount of a compound of formula (I) or a pharmaceutically acceptable active ester thereof.

The following Examples illustrate the invention.

EXAMPLE 1

a) E-3-methyl-5-(2-bromovinyl)-2'-deoxyuridine

To a solution of E-5-(2-bromovinyl)-2'-deoxyuridine (666 mg, 2 mmol) in anhydrous tetrahydrofuran (20 mL), containing tetra-n-butylammonium fluoride (1M in THF, 2 mL), was added iodomethane (10 mL) and the resultant mixture stirred at 25°C overnight.

The tetrahydrofuran and excess iodomethane were removed under reduced pressure and the residue subjected to short-path column chromatography on silica gel using 10-30% acetone in n-hexane as eluent. Pure E-3-methyl-5-(2-bromovinyl)-2'-deoxyuridine was isolated in 32% yield with mp 161°C.

$\nu$ max (KBr) 3470, 3063, 1690, 1640, 1625, 1480, 1096 $cm^{-1}$;

$^1H$ nmr $[(CD_3)_2SO]$ $\delta$2.18 (2H, m, 2'-$CH_2$), 3.23 (3H, s, 3-$CH_3$), 3.60 (2H, m, 5'-$CH_2$), 3.82 (1H, m, 4'-CH), 4.25 (1H, m, 3'-CH), 5.05 (1H, d, 5'-OH, $D_2O$ exchangeable), 5.20 (1H, t, 3'-OH, $D_2O$ exchangeable), 6.15 (1H, t, 1'-CH), 6.82 (1H, d, J=13.3Hz, CH=CHBr), 7.25 (1H, d, J=13.3Hz, CH=CHBr), 8.12 (1H, s, 6-CH);

m/e (70eV) 347 ($M^+$, 7%).

Analysis

Found: C 41.69; H 4.39; N 7.84%. $C_{12}H_{15}N_2O_5Br$:
requires: C 41.49; H 4.32; N 8.06%.

b) Alternative process for synthesis of E-3-methyl-5-(2-bromovinyl)-2'-deoxyuridine

A mixture of E-5-(2-bromovinyl)-2'-deoxyuridine (666 mg, 2 mmol) and N,N-dimethylformamide dimethylacetal (3 mL, 22.5 mmol) was heated to boiling under a nitrogen atmosphere and trifluoroacetic acid (0.02 mL) introduced cautiously through a serum cap. The mixture was heated under reflux for an additional 15 hr and then evaporated to dryness. Column chromatography on silica gel afforded pure product in 56% yield identical in every respect to that prepared by process (a).

## EXAMPLE 2

### E-5-(2-Chlorovinyl)-3-methyl-2'-deoxyuridine

To E-5-(2-chlorovinyl)-2'-deoxyuridine (2.02g, 7mmol) in dimethylformamide dimethylacetal (10mL), trifluoroacetic acid (0.1mL) was added and the mixture heated under reflux for 19 hours with exclusion of atmospheric moisture. Removal of the solvent yielded a brown gum which was purified by column chromatography on silica gel eluting with chloroform-methanol mixtures. E-5-(2-Chlorovinyl)-2'-deoxyuridine was obtained as a crystalline solid (1.4g, 66%), m.p. 138-139°;

$\nu_{max}$ (methanol) 247.5 nm ($\varepsilon$ 14,300), 291 nm ($\varepsilon$ 10,700); $\nu_{max}$ (KBr) 3480, 1700, 1652, 1605, 1480 $cm^{-1}$;

$^1$Hnmr [$(CD_3)_2SO$] $\delta$ 2.16 (2H, t, J = 6Hz, 2'-$CH_2$), 3.18 (3H, s, 3-$CH_3$), 3.60 (2H, m, 5'-$CH_2$), 3.78 (1H, m, 4'-CH), 4.23 (1H, m, 3'-CH), 5.07 (1H, t, J = 5Hz, 5'-OH), 5.22 (1H, d, J = 4Hz, 3'-OH), 6.11 (1H, t, J = 6Hz, 1'-CH), 6.59 (1H, d, J= 13Hz, C<u>H</u>=CHCl), 7.19 (1H, d, J = 13Hz, CH=C<u>H</u>Cl), 8.12 (1H, s, 6-CH).

M/e observed 302.0672 ($M^+$ theorectical 302.0667).

### Analysis

Found: C, 47.58; H, 4.99; N, 9.43% $C_{12}H_{15}ClN_2O_5$
requires: C, 47.60; H, 4.96; N, 9.26%

## DEMONSTRATION OF EFFECTIVENESS OF THE COMPOUND OF EXAMPLE 1

### 1. In Cell Culture

Vero (African Green Monkey Kidney) cells were grown to confluence in 6 well multidishes, each well being 3.5 cm in diameter. The cells were incubated with Herpes simplex type 1 virus (HFEM strain) and overlaid with 0.5 ml of 0.9% agarose (w/v) in maintenance medium containing the test compound at a range of concentrations from 100 μg/mL in half-log dilution steps. The virus infected cultures were then incubated at 37°C for 6 days before fixing in 4% formaldehyde solution and staining with carbolfuchsin. The dishes were then examined to find what concentration of test compound causing a 50% reduction in the number of virus plaques formed ($PDD_{50}$ value) and the minimum concentration of test compound which killed the cell monolayer, leaving a clear zone devoid of cells and virus plaques (MTD).

### Results

| Compound | $PDD_{50}$ (μg/mL) | MTD (μg/mL) |
|---|---|---|
| 3-Methyl-*E*-5-(2-bromovinyl)-2'-deoxyuridine | 5.4 | >100 |

2. In Vivo Blood Level Studies

Single 100 mg/kg doses of the compound were administered orally as suspensions in carboxymethyl cellulose to groups of 3 starved female Balb/c mice. Blood was collected 120 minutes after dosing and the serum separated and stored at -20°C. Heat inactivated sera from single mice were titrated in plaque inhibition assays using the KOS strain of Herpes simplex type 1 virus in Vero cell monolayers. Antiviral activity is calculated by extrapolation from a standard inhibition dose response curve for E-5-(2-bromovinyl)-2'-deoxyuridine and is quoted as the equivalent concentration of E-5-(2-bromovinyl)-2'-deoxyuridine (in µg/mL) giving the same antiviral activity in cell culture.

Results

| Time after dosing (minutes) | 3-Methyl-E-5-(2-bromovinyl)-2'-deoxyuridine | E-5-(2-bromovinyl)-2'-deoxyuridine |
|---|---|---|
| 120 | 3.1 ± 0.8 | 1.7 ± 0.5 |

The above results show that, 120 minutes after dosing, the blood level of antiviral activity in the compound of Example 1 is nearly twice that of the potent antiviral agent E-5-(2-bromovinyl)-2'-deoxyuridine.

CLAIMS

1. A compound of formula (I), or a pharmaceutically acceptable active ester thereof:

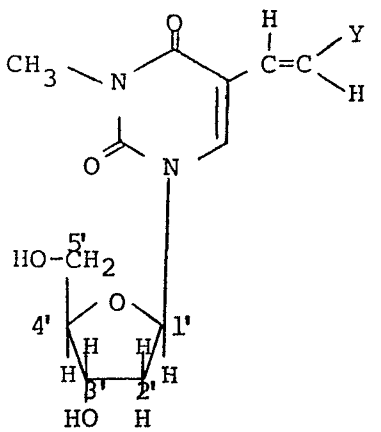

(I)

in which Y is a halogen atom.

2. A compound according to claim 1, in which Y is a bromine atom.

3. A compound according to claim 1, selected from E-3-methyl-5-(2-bromovinyl)-2'-deoxyuridine, and E-5-(2-Chlorovinyl)-3-methyl-2'-deoxyuridine.

4. A process for preparing a compound according to claim 1, which comprises treating a compound of formula (II):

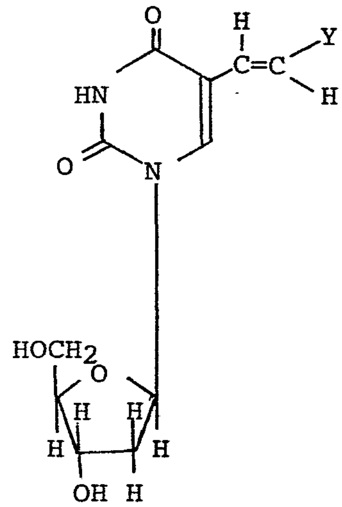

(II)

in which Y is as defined in formula (I), with a methyl halide of formula $CH_3X$, in which X is a halogen atom, or with N,N-dimethylformamide dimethylacetal.

5. A pharmaceutical composition comprising a compound according to any one of claims 1 to 3, together with a pharmaceutically acceptable carrier or excipient.

6. A compound according to any one of claims 1 to 3, or a composition according to claim 5, for use in the treatment of viral infections.

0072137

European Patent Office

# EUROPEAN SEARCH REPORT

Application number

EP 82 30 3933

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| | --- | | |
| Y | DE-A-2 915 254 (UNIVERSITY OF BIRMINGHAM) * pages 1-2 * & GB - A - 1 601 020 (Cat.D) | 1,5 | C 07 H 19/06<br>A 61 K 31/70 |
| | --- | | |
| Y | DE-A-1 620 185 (ROBUGEN) * page 1 * & GB - A - 1 750 565 | 1,5 | |
| | ----- | | |
| | | | TECHNICAL FIELDS SEARCHED (Int. Cl. 3) |
| | | | C 07 H 19/00<br>A 61 K 31/00 |

The present search report has been drawn up for all claims

| Place of search | Date of completion of the search | Examiner |
|---|---|---|
| THE HAGUE | 04-11-1982 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document